# EUROPEAN PATENT APPLICATION

(11) **EP 3 748 649 A1**
(43) Date of publication of application: **09.12.2020**
(21) Application number: 19897520.3
(22) Date of filing: 11.11.2019
(51) Int. Cl.: G16H 50/30

(54) **METHOD FOR EVALUATING MULTI-MODAL EMOTIONAL UNDERSTANDING CAPABILITY OF PATIENT WITH AUTISM SPECTRUM DISORDER**

(30) Priority: 14.12.2018 CN 201811531585
(71) Applicant: Shenzhen Institutes of Advanced Technology, Shenzhen, Guangdong 518055 (CN)
(72) Inventor: WANG, Lan, Shenzhen, Guangdong 518055 (CN); YAN, Nan, Shenzhen, Guangdong 518055 (CN); WANG, JianYing, Shenzhen, Guangdong 518055 (CN); SU, Qi, Shenzhen, Guangdong 518055 (CN)
(74) Representative: Fabry, Bernd
(86) International application number: PCT/CN2019/117032
(87) International publication number: WO 2020/119355

(57) **Abstract**

The present disclosure relates to a method for evaluating a multi-modal emotion cognition capability of a patient with an autism spectrum disorder, mainly including the following steps: (1) setting a multi-modal evaluation environment, where the multi-modal evaluation environment is used to stimulate a tested object; and the multi-modal evaluation environment includes: a visual scene, a verbal communication scene, and an emotional communication scene; (2) collecting multi-modal evaluation information, where the evaluation information includes: intelligence information, behavioral data, and eye movement data of the tested object; and (3) performing statistical analysis, to obtain an emotion cognition capability of the tested object, where the performing statistical analysis includes: performing statistical analysis on the behavioral data, performing statistical analysis on the eye movement data, and performing statistical analysis on a combination of the behavioral data and the eye movement data. Based on the method for evaluating a multi-modal emotion cognition capability of a patient with an autism spectrum disorder in the present disclosure, an objective evaluation approach is added in addition to an original manual evaluation, to provide evidences for accurately and effectively evaluating a social communication capability and an emotion cognition capability of the patient.

## Description

### TECHNICAL FIELD

The present disclosure relates to a method for evaluating a multi-modal emotion cognition capability of a patient with an autism spectrum disorder based on an eye movement technology.

### BACKGROUND ART

In recent years, in research on an autism spectrum disorder, it is generally believed that patients with autism spectrum disorders have characteristics such as stiff behavior and social disorders, and the social disorders are closely related to emotional perception. The social disorders cause patients with autism spectrum disorders to have certain emotional cognition defects, which in turn exacerbates the social disorders of patients with autism spectrum disorders. Therefore, more and more research has begun to focus on a social communication capability and an emotion recognition capability of patients with autism spectrum disorders.

At present, a most common evaluation method is a manual evaluation method. A manual evaluation process takes a long time and requires a relatively high capability of doctors. During evaluation, due to cooperation degrees of patients and individual differences, patients often do not cooperate and it is difficult to score. In addition, because patients with autism spectrum disorders are heterogeneous, they cannot be diagnosed through a certain examination, and can only be diagnosed based on a symptomatic characteristic, medical history data, and a social function. Therefore, there are many subjective factors and uncontrollable factors in evaluation results.

### SUMMARY

To resolve the problems existing in the background art, the present disclosure provides a method for evaluating a multi-modal emotion cognition capability of a patient with an autism spectrum disorder, and an objective evaluation approach is added in addition to an original manual evaluation, to provide evidences for accurately and effectively evaluating a social communication capability and an emotion cognition capability of the patient.

A technical solution of the present disclosure to resolving the foregoing problem is as follows: a method for evaluating a multi-modal emotion cognition capability of a patient with an autism spectrum disorder is provided, including the following steps:
(1) setting a multi-modal evaluation environment, where
   the multi-modal evaluation environment is used to stimulate a tested object; and
   the multi-modal evaluation environment includes: a visual scene, a verbal communication scene, and an emotional communication scene;
(2) collecting multi-modal evaluation information, where
   the evaluation information includes: intelligence information, behavioral data, and eye movement data of the tested object; and
(3) performing statistical analysis, to obtain an emotion cognition capability of the tested object, where
   the performing statistical analysis includes: performing statistical analysis on the behavioral data, performing statistical analysis on the eye movement data, and performing statistical analysis on a combination of the behavioral data and the eye movement data.

Further, in step (1),
the visual scene includes a real scene and/or a virtual scene; in the real scene, a life-based dynamic video is used as a stimulus material, so that a real emotion of a character in real life can be shown; in the virtual scene, a head portrait of an automatically composited flexible three-dimensional virtual talker is used as a stimulus material, so that a real-time and dynamic human-robot verbal expression and communication with a corresponding facial expression can be truly simulated in a human-robot interaction process.

Further, in step (1),
the verbal communication scene includes a single person talking scene and/or a multi-person communication scene; in the single person talking scene, a video in which only one person appears is used as a stimulus material, description is provided in a close-up, and a facial expression of a character can be subtly shown; in the multi-person communication scene, a video in which verbal communication and emotion expressions among a plurality of persons appear is used as a stimulus material, which can reflect that emotional communication between persons is performed through verbal communication or interaction.

Further, in step (1),
the emotional communication scene includes a single-sentence scene and/or a dialogue scene; in the single-sentence scene, sentences of different stimulus materials in a video material are independent of each other, and an inner emotion of a character can be reflected by saying only one sentence; in the dialogue scene, there are dialogues and contexts between characters in the video material, and different emotions are reflected based on different situations.

Further, in step (2),
the collecting intelligence information is specifically: recording basic information about gender, an actual age, a psychological age, and a verbal capability of the tested object or a guardian of the tested object through inquiry, and obtaining intelligence information and cognitive information through intelligence evaluation the like.

Further, in step (2),
the collecting behavioral data is specifically: recording, by using an audio/video collection device in an entire process, a behavior action of the patient with an autism spectrum disorder when the tested object performs emotion recognition in different scenes, including special behavior such as self-talk, an emotional loss of control, and repetitive words, and recording a judgment made when the patient with an autism spectrum disorder performs emotion recognition.

Further, in step (2),
the collecting eye movement data is specifically: collecting eye movement data when the tested object watches a video material, where the eye movement data includes a focus map, a heat map, an eye movement trajectory, and a pupil size.

Further, in step (3),
the performing statistical analysis on the behavioral data is specifically: collecting statistics about an emotion cognition judgment accuracy rate of the patient with an autism spectrum disorder with a different emotion based on the judgment made when the patient with an autism spectrum disorder performs emotion recognition; analyzing, through statistical analysis, a difference when the patient with an autism spectrum disorder watches a positive emotion and a negative emotion; and analyzing a correlation between the intelligence information and the emotion cognition judgment accuracy rate of the patient with an autism spectrum disorder through correlation analysis.

Further, in step (3),
the performing statistical analysis on the eye movement data is: calculating, based on the collected eye movement data, entering time, fixation time, and a fixation point quantity when the patient with an autism spectrum disorder watches an area of interest (such as an eye, a mouth, or a face) in a video stimulus material; analyzing a difference between entering time, a difference between fixation time, and a difference between fixation point quantities for the patient with an autism spectrum disorder in different areas of interest through statistical analysis; and analyzing, through correlation analysis, a correlation between the intelligence information of the patient with an autism spectrum disorder and entering time, fixation time, as well as fixation point quantities for the patient with an autism spectrum disorder in different areas of interest.

Further, in step (3),
the performing statistical analysis on a combination of the behavioral data and the eye movement data is specifically: analyzing, through statistical analysis, a difference between the emotion cognition judgment accuracy rate, and the entering time, the fixation time, and the fixation point quantities in the different areas of interest based on the emotion cognition judgment accuracy rate that is of the patient with an autism spectrum disorder with different emotions and whose statistics in the behavioral data are collected, and based on physiological eye movement data such as the entering time, the fixation time, and the fixation point quantities for the patient with an autism spectrum disorder in the different areas of interest; and analyzing a correlation between the emotion cognition judgment accuracy rate, and the entering time, the fixation time, and the fixation point quantities in the different areas of interest through correlation analysis.

Advantages of the present disclosure are as follows:
(1) There are a plurality of designs of stimulus materials, such as a real scene, a virtual scene, a multi-person scene, a single person scene, a single sentence scene, and a contextual scene, and the different emotional intensities of character faces in the foregoing various types of scenes are adjusted. A social cognition capability and an emotion recognition capability of the patient with an autism spectrum disorder are comprehensively evaluated. From different perspectives, it can be learned that the patient with an autism spectrum disorder has defects in many aspects of emotion recognition or has an obvious defect in an aspect of emotion recognition.
(2) Since eye movement tracking is non-invasive, an evaluated person does not need to wear any apparatus. During evaluation, the evaluated person does not give up the evaluation due to discomforts. In addition, recorded physiological data is a response of the evaluated person in a natural state, and can more truly reflect social skills of the evaluated person.
(3) Subjective and objective evaluations are combined, and an objective statistical analysis method is used to analyze the physiological data of the patient with an autism spectrum disorder. Compared with a traditional evaluation scale, the present disclosure has a simple procedure, is easy to operate, has a short period, is not limited by capabilities and experience of doctors, and a local medical level, and can be used as evaluation criteria for evaluation.
(4) To perform fusion evaluation on multi-modal information of eye movement data and behavioral data, a social capability of the patient with an autism spectrum disorder can be comprehensively evaluated by making full use of advantages shown when evaluation is separately performed based on the eye movement data and the behavioral data, so that the evaluation method is reliable, and the results are accurate.
(5) The evaluation results of social cognition and emotion recognition of patients with autism spectrum disorders show that a subjective cognition judgment accuracy rate is significantly lower than that of a normal development control group, and less attention is paid to core areas such as an eye and a face for emotion recognition. A psychological age of the patient with an autism spectrum disorder is positively correlated with the subjective cognition judgment accuracy rate, and a fixation point and fixation time for the facial area are positively correlated with the psychological age.

### BRIEF DESCRIPTION OF THE DRAWINGS

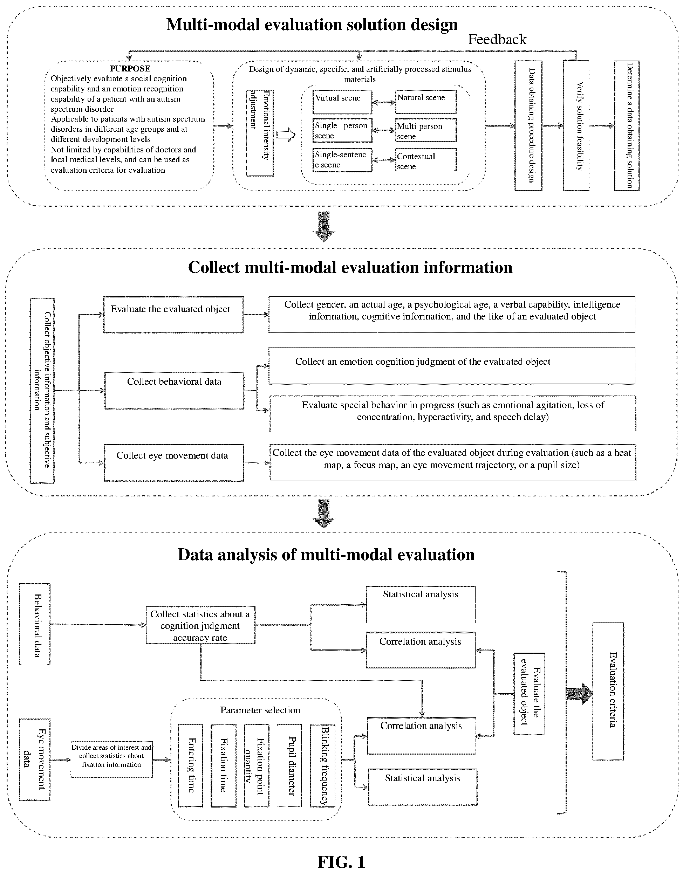

FIG. 1 is a flowchart of a method for evaluating a multi-modal emotion cognition capability of a patient with an autism spectrum disorder according to the present disclosure.

### DETAILED DESCRIPTION

In order to make objectives, technical solutions, and advantages of implementations of the present disclosure clearer, the technical solutions in the implementations of the present disclosure are described clearly and completely below with reference to accompanying drawings in the implementations of the present disclosure. Apparently, the described implementations are only some rather than all of the implementations of the present disclosure. All other implementations obtained by persons of ordinary skill in the art based on the implementations of the present disclosure without creative efforts shall fall within the protection scope of the present disclosure. Therefore, the detailed description on the implementations of the present disclosure in the accompanying drawings is not intended to limit the protection scope of the present disclosure, and are merely selected implementations of the present disclosure. All other implementations obtained by persons of ordinary skill in the art based on the implementations of the present disclosure without creative efforts shall fall within the protection scope of the present disclosure.

A method for evaluating a multi-modal emotion cognition capability of a patient with an autism spectrum disorder is provided, including the following steps:
(1) A multi-modal evaluation environment is set, where
   the multi-modal evaluation environment is used to stimulate a tested object; and
   the multi-modal evaluation environment includes: a visual scene, a verbal communication scene, and an emotional communication scene.
   In order to objectively evaluate a social cognition capability and an emotion recognition capability of the patient with an autism spectrum disorder, and to apply to different age groups, different development levels, and limitations by local medical levels, even if a doctor is not competent or experienced, the doctor can still accurately evaluate the patient with an autism spectrum disorder, during design of stimulus materials, the present disclosure uses dynamic, specific, and artificially processed scenes as the stimulus materials, specifically:
   In step (1), the visual scene includes a real scene and/or a virtual scene; in the real scene, a life-based dynamic video is used as a stimulus material, so that a real emotion of a character in real life can be shown; in the virtual scene, a head portrait of an automatically composited flexible three-dimensional virtual talker is used as a stimulus material, so that a real-time and dynamic human-robot verbal expression and communication with a corresponding facial expression can be truly simulated in a human-robot interaction process. the patient with an autism spectrum disorder cannot make eye contact with others like ordinary people. However, in a created virtual scene, the patient with an autism spectrum disorder can get practiced through interaction with a virtual character, to explore whether the simulated and relatively simple virtual scene relative to a real scene can provide more effective information in a process of emotion recognition of the patient with an autism spectrum disorder.
   In step (1), the verbal communication scene includes a single person talking scene and/or a multi-person communication scene; in the single person talking scene, a video in which only one person appears is used as a stimulus material, description is provided in a close-up, and a facial expression of a character can be subtly shown; in the multi-person communication scene, a video in which verbal communication and emotion expressions among a plurality of persons appear is used as a stimulus material, which can reflect that emotional communication between persons is performed through verbal communication or interaction. The types of scenes are designed as the stimulus materials to explore a similarity and a difference between emotion recognition capabilities and social communication capabilities of the patient with an autism spectrum disorder in two scenes: the single person scene and the multi-person scene.
   In step (1), the emotional communication scene includes a single-sentence scene and/or a dialogue scene; in the single-sentence scene, sentences of different stimulus materials in a video material are independent of each other, and an inner emotion of a character can be reflected by saying only one sentence; in the dialogue scene, there are dialogues and contexts between characters in the video material, and different emotions are reflected based on different situations. The types of scenes are designed as the stimulus materials to explore a capability of the patient with an autism spectrum disorder to understand content of words and a capability to understand an emotion expressed in each sentence in different situations.
   Based on the above various scenes in the present disclosure, different expression intensities of characters in the scenes are adjusted, among which the expression intensities are classified into three intensity levels: a low intensity, a medium intensity, and a high intensity. Videos with different expression intensities are used as stimulus materials. Impact of different expression intensities on the patient with an autism spectrum disorder is analyzed, and the patient with an autism spectrum disorder can be more comprehensively evaluated based on the stimulus materials with different intensities, so that a specific defect aspect of the patient with an autism spectrum disorder can be more accurately learned, thereby facilitating further refinement of the evaluation.
   Based on the present disclosure, the different stimulus materials are designed to comprehensively evaluate the social cognition capability and the emotion recognition capability of the patient with an autism spectrum disorder. From different perspectives, it can be seen that the patient with an autism spectrum disorder has defects in many aspects of emotion recognition or has an obvious defect in an aspect of emotion recognition. After a data obtaining solution is preliminarily designed, in the present disclosure, a preliminary experiment for data obtaining is conducted. The experimental solution and the design of the stimulus materials are further adjusted based on result analysis of the preliminary experiment, and finally the experimental solution is formed.
(2) Multi-modal evaluation information is collected.
   The present disclosure aims to collect subjective data and objective data of the patient with an autism spectrum disorder. The subjective data and the objective data are collected in the following three aspects:
   Based on the present disclosure, the collecting intelligence information of the patient with an autism spectrum disorder is as follows: recording some basic information about gender, an actual age, a psychological age, a verbal capability, and the like of the patient with an autism spectrum disorder or a guardian of the patient with an autism spectrum disorder through inquiry, and obtaining intelligence information, cognitive information, and the like of the patient with an autism spectrum disorder through intelligence evaluation.
   Based on the present disclosure, the collecting behavioral data of the patient with an autism spectrum disorder is as follows: recording, by using an audio/video collection device in an entire process, a behavior action of the patient with an autism spectrum disorder when the patient with an autism spectrum disorder performs emotion recognition in different scenes, including some specific behavior such as self-talk, an emotional loss of control, and repetitive words, and recording a judgment made when the patient with an autism spectrum disorder performs emotion recognition.
   Based on the present disclosure, the collecting eye movement data of the patient with an autism spectrum disorder is as follows: recording fixation information of the patient with an autism spectrum disorder in real time by using an eye movement tracking technology. An experiment is conducted in a separate and quiet room. Before a formal experiment begins, eyes of the patient with an autism spectrum disorder are level with the center of a screen and a distance is about 60 cm. Calibration is carried out before each experiment, and the formal experiment can be conducted only after the calibration succeeds. During the formal experiment, a series of video stimulus materials in a scene are presented on a tested screen. The patient with an autism spectrum disorder is required to watch the video stimulus material. Each time after completing watching a video stimulus material, the patient with an autism spectrum disorder needs to judge an emotion expressed by the video stimulus material, and select a button labeled as the corresponding emotion. An eye tracker collects eye movement data of the tested object while watching the video material, including a focus map, a heat map, an eye movement trajectory, a pupil size, and the like.
(3) Statistical analysis is performed, to obtain an emotion cognition capability of the tested object.

The present disclosure aims to combine the subjective data and the objective data to comprehensively evaluate the social cognition capability and the emotion recognition capability of the patient with an autism spectrum disorder. Data analysis is mainly performed in the following three aspects:
1. Statistical analysis is performed on the behavioral data: collecting statistics about an emotion cognition judgment accuracy rate of the patient with an autism spectrum disorder with different emotions based on the judgment made when the patient with an autism spectrum disorder performs emotion recognition; analyzing, through statistical analysis, a difference when the patient with an autism spectrum disorder watches a positive emotion and a negative emotion; and analyzing a correlation between the intelligence information and the emotion cognition judgment accuracy rate of the patient with an autism spectrum disorder through correlation analysis.
2. Statistical analysis is performed on the eye movement data: calculating, based on the collected eye movement data, entering time, fixation time, and a fixation point quantity when the patient with an autism spectrum disorder watches an area of interest (such as an eye, a mouth, or a face) in a video stimulus material; analyzing a difference between entering time, a difference between fixation time, a difference between fixation point quantities, and the like for the patient with an autism spectrum disorder in different areas of interest through statistical analysis; and analyzing, through correlation analysis, a correlation between the intelligence information of the patient with an autism spectrum disorder and the entering time, the fixation time, the fixation point quantities, and the like for the patient with an autism spectrum disorder in different areas of interest.
3. Statistical analysis is performed on a combination of the behavioral data and the eye movement data: analyzing, through statistical analysis, a difference between the emotion cognition judgment accuracy rate, and the entering time, the fixation time, the fixation point quantities, and the like in the different areas of interest based on the emotion cognition judgment accuracy rate that is of the patient with an autism spectrum disorder with different emotions and whose statistics in the behavioral data are collected, and based on physiological eye movement data such as the entering time, the fixation time, and the fixation point quantities for the patient with an autism spectrum disorder in the different areas of interest; and analyzing a correlation between the emotion cognition judgment accuracy rate, and the entering time, the fixation time, the fixation point quantities, and the like in the different areas of interest through correlation analysis

In the evaluation process of the patient with an autism spectrum disorder, the intelligence information, the behavioral data, and the eye movement data of patients with autism spectrum disorders are integrated, and a large quantity of data analysis is performed to draw rules for evaluation. In a case of unbalanced medical development in various regions and limitations on medical development, the present disclosure can be used as evaluation criteria for evaluation.

The present disclosure is verified by three experiments.

In a first experiment, a life-based dynamic video is used to evaluate and test a child patient with a moderately functional autism spectrum disorder. An experimental result shows an emotion recognition disorder of the patient with an autism spectrum disorder and a difference of physiological eye movement data from a normal control group. Compared with the normal control group, the patient with an autism spectrum disorder has significantly less fixation and interest in eyes, a mouth, and another area of a face, and prefers a background that is of social cognition and emotion recognition and that has no social significance. In addition, the patient with an autism spectrum disorder has different manifestation on different types of emotions. The patient with an autism spectrum disorder is better at cognition of a positive emotion, and also needs more attention to interpret a negative emotion.

In a second experiment, a life-based dynamic video is used to evaluate and test a child patient with a highly functional autism spectrum disorder. In addition, reliability and universality of this patent is explored by adding a Carroll intelligence evaluation. An experimental result shows that the patient with an autism spectrum disorder has defects in social cognition and emotion recognition, and a subjective cognition judgment accuracy rate is significantly lower than that of the control group. Eye movement data analysis shows that the patient with an autism spectrum disorder pays less attention to certain core areas (an eye or a face) for emotion recognition in an emotion recognition process than a typical development tested object, but to a mouth, there is no attention difference. In addition, age of the patient with an autism spectrum disorder (a score of the Carroll intelligence evaluation) is positively correlated with the subjective cognition judgment accuracy rate. The fixation point and fixation time in the facial area are positively correlated with the psychological age. A higher psychological age score leads to a higher judgment accuracy rate and a longer fixation time and more fixation points in the face. In this way, reliability of the present disclosure is once again proved.

In a third experiment, a dynamic video in which a head portrait of a three-dimensional virtual talker has a contextual dialogue and different emotions with intensity changes is used to evaluate and test a child patient with an autism spectrum disorder. The head portrait of the three-dimensional virtual talker is automatically composited and flexible, is a real-time, dynamic, and three-dimensional facial emotion expression system with a verbal communication function, and can truly simulate human-robot emotional communication with verbal and facial expressions in a human-robot interaction process. Physiological eye movement data when a patient with an autism disorder watches the dynamic video in which the head portrait of the three-dimensional virtual talker has a contextual dialogue and intensity changes is collected. Through mathematical statistical analysis, a difference between fixation manners of the patient with an autism disorder and a normal control group when the patient with an autism disorder and the normal control group watch the video of the head portrait of the 3D virtual talker, as well as a relationship between a subjective cognition judgment accuracy rate of the patient with an autism disorder and an emotional intensity change of the dynamic head portrait of the 3D virtual talker are analyzed and compared, to obtain emotional cognition of the patient with an autism disorder on the dynamic head portrait of the 3D virtual talker with a contextual dialogue and intensity changes, so as to rationally evaluate a social cognition capability and an emotional cognition degree of the patient with an autism disorder.

The foregoing is embodiments of the present disclosure and does not constitute a limitation on the protection scope of the patent of the present disclosure. Any equivalent structure or equivalent procedure change made by using the description and the accompanying drawings of the present disclosure, or direct or indirect application thereof in other related technical fields, shall still fall within the protection scope of the patent of the present disclosure.

## Claims

1. A method for evaluating a multi-modal emotion cognition capability of a patient with an autism spectrum disorder, comprising the following steps:
(1) setting a multi-modal evaluation environment, wherein
the multi-modal evaluation environment is used to stimulate a tested object; and
the multi-modal evaluation environment comprises: a visual scene, a verbal communication scene, and an emotional communication scene;
(2) collecting multi-modal evaluation information, wherein
the evaluation information comprises: intelligence information, behavioral data, and eye movement data of the tested object; and
(3) performing statistical analysis, to obtain an emotion cognition capability of the tested object, wherein
the performing statistical analysis comprises: performing statistical analysis on the behavioral data, performing statistical analysis on the eye movement data, and performing statistical analysis on a combination of the behavioral data and the eye movement data.

2. The method for evaluating the multi-modal emotion cognition capability of the patient with the autism spectrum disorder according to claim 1, wherein in step (1),
the visual scene comprises a real scene and/or a virtual scene; in the real scene, a life-based dynamic video is used as a stimulus material, to show a real emotion of a character in real life; in the virtual scene, a head portrait of an automatically composited three-dimensional virtual talker is used as a stimulus material, so that a real-time and dynamic human-robot verbal expression and communication with a corresponding facial expression can be truly simulated in a human-robot interaction process.

3. The method for evaluating the multi-modal emotion cognition capability of the patient with the autism spectrum disorder according to claim 1, wherein in step (1),
the verbal communication scene comprises a single person talking scene and/or a multi-person communication scene; in the single person talking scene, a video in which only one person appears is used as a stimulus material, description is provided in a close-up, and a facial expression of a character can be subtly shown; in the multi-person communication scene, a video in which verbal communication and emotion expressions among a plurality of persons appear is used as a stimulus material, which can reflect that emotional communication between persons is performed through verbal communication or interaction.

4. The method for evaluating the multi-modal emotion cognition capability of the patient with the autism spectrum disorder according to claim 1, wherein in step (1),
the emotional communication scene comprises a single-sentence scene and/or a dialogue scene; in the single-sentence scene, sentences of different video stimulus materials are independent of each other, and an inner emotion of a character can be reflected by saying only one sentence; in the dialogue scene, there are dialogues and contexts between characters in a video material, and different emotions are reflected based on different situations.

5. The method for evaluating the multi-modal emotion cognition capability of the patient with the autism spectrum disorder according to any one of claims 1 to 4, wherein in step (2), collecting the intelligence information is specifically: recording basic information about gender, an actual age, a psychological age, and a verbal capability of the tested object or a guardian of the tested object through inquiry, and obtaining intelligence information and cognitive information through intelligence evaluation.

6. The method for evaluating the multi-modal emotion cognition capability of the patient with the autism spectrum disorder according to claim 5, wherein in step (2),
collecting the behavioral data is specifically: recording, by using an audio/video collection device in an entire process, a behavior action of the tested object when the tested object performs emotion recognition in different scenes, comprising self-talk, an emotional loss of control, and repetitive words, and recording a judgment made when the tested object performs emotion recognition.

7. The method for evaluating the multi-modal emotion cognition capability of the patient with the autism spectrum disorder according to claim 6, wherein in step (2),
collecting the eye movement data is specifically: collecting eye movement data when the tested object watches a video material, wherein the eye movement data comprises a focus map, a heat map, an eye movement trajectory, and a pupil size.

8. The method for evaluating the multi-modal emotion cognition capability of the patient with the autism spectrum disorder according to claim 7, wherein in step (3),
the performing statistical analysis on the behavioral data is specifically: collecting statistics about an emotion cognition judgment accuracy rate of the tested object with a different emotion based on the judgment made when the tested object performs emotion recognition; analyzing, through statistical analysis, a difference when the tested object watches a positive emotion and a negative emotion; and analyzing a correlation between the intelligence information and the emotion cognition judgment accuracy rate of the tested object through correlation analysis.

9. The method for evaluating the multi-modal emotion cognition capability of the patient with the autism spectrum disorder according to claim 8, wherein in step (3),
performing the statistical analysis on the eye movement data is: calculating, based on the collected eye movement data, entering time, fixation time, and a fixation point quantity when the tested object watches an area of interest in a video stimulus material; analyzing a difference between entering time, a difference between fixation time, and a difference between fixation point quantities for the tested object in different areas of interest through statistical analysis; and analyzing, through correlation analysis, a correlation between the intelligence information of the tested object and entering time, fixation time, as well as fixation point quantities for the tested object in different areas of interest.

10. The method for evaluating the multi-modal emotion cognition capability of the patient with the autism spectrum disorder according to claim 9, wherein in step (3),
performing the statistical analysis on a combination of the behavioral data and the eye movement data is specifically: analyzing, through statistical analysis, a difference between the emotion cognition judgment accuracy rate, and the entering time, the fixation time, as well as the fixation point quantities in the different areas of interest based on the emotion cognition judgment accuracy rate that is of the tested object with different emotions and whose statistics in the behavioral data are collected, and based on physiological eye movement data of the entering time, the fixation time, and the fixation point quantities for the tested object in the different areas of interest; and analyzing a correlation between the emotion cognition judgment accuracy rate, and the entering time, the fixation time, as well as the fixation point quantities in the different areas of interest through correlation analysis.
